# EUROPÄISCHE PATENTSCHRIFT

(11) **EP 3 296 075 B1**
(45) Veröffentlichungstag und Bekanntmachung des Hinweises auf die Patenterteilung: **10.11.2021**
(21) Anmeldenummer: 16189665.9
(22) Anmeldetag: 20.09.2016
(51) Int. Cl.: B29B 7/28, B29B 7/76, B29C 31/06, B29C 67/24, G01N 7/00

(54) **FÖRDERVORRICHTUNG ZUM FÖRDERN EINES VISKOSEN MATERIALS AUS EINEM BEHÄLTER UND VERFAHREN ZUM BETREIBEN DER FÖRDERVORRICHTUNG**
FEEDING APPARATUS FOR FEEDING A VISCOUS MATERIAL FROM A CONTAINER AND METHOD FOR OPERATING THE FEEDING APPARATUS
DISPOSITIF DE TRANSPORT D'UNE MATIÈRE VISQUEUSE PROVENANT D'UN RÉCIPIENT ET PROCÉDÉ DE FONCTIONNEMENT DU DISPOSITIF DE TRANSPORT

(43) Veröffentlichungstag der Anmeldung: 21.03.2018
(73) Patentinhaber: WAGNER INTERNATIONAL AG, 9450 Altstätten (CH)
(72) Erfinder: Gerke, Thomas, 35708 Haiger (DE); Naber, Marcus, 42799 Leichlingen (DE); Schygulla, Christian, 58540 Meinerzhagen (DE)
(74) Vertreter: Nückel, Thomas

(56) Entgegenhaltungen:
- EP-A2- 0 451 752
- WO-A2-2009/140776
- DE-A1- 3 803 594
- DE-A1-102004 038 801
- DE-U1- 29 712 263
- DE-U1-202011 108 222
- GB-A- 2 329 257
- US-A- 4 376 172
- US-A- 5 257 723
- US-A1- 2014 326 322

## Beschreibung

### Technisches Gebiet

Die Erfindung betrifft eine Fördervorrichtung zum Fördern von viskosen Materialien aus einem Behälter und ein Verfahren zum Betreiben der Fördervorrichtung.

Mit der Fördervorrichtung können zuverlässig verschiedene mittel- bis hochviskose Materialien, wie zum Beispiel Dichtmittel, Klebstoffe oder Silikonkautschuk, aus Behältern, wie zum Beispiel Eimern oder Fässern, zu verschiedenen Verarbeitungssystemen gefördert werden. Die Fördervorrichtung wird auch als Zufuhrsystem zum Transport von viskosen Materialien bezeichnet.

Zur Produktion von Spritzgussteilen aus einem viskosen Einkomponenten- oder Mehrkomponenten-Material, beispielsweise Flüssig-Silikon, auch Liquid Silicone Rubber (LSR) genannt, können Spritzgiessmaschinen verwendet werden. Das viskose Material wird vom Materiallieferant in der Regel in Behältern mit 20 l bis 200 l zur Verfügung gestellt. Mittels einer Fördervorrichtung wird das Material aus dem Behälter herausgefördert und der Spritzgiessmaschine zugeführt. Wenn Mehrkomponenten-Material benutzt wird, kann zudem eine Mischvorrichtung vorgesehen sein, mit der die einzelnen Komponenten gemischt und dann der der Spritzgiessmaschine zugeführt werden.

### Stand der Technik

Aus dem Druckschrift WO 2014/056011 A2 ist eine solche Förder- und Mischvorrichtung zum Mischen von zwei Flüssigkeiten bekannt. Die Förder- und Mischvorrichtung umfasst eine Pumpe, die mit einer Folgeplatte verbunden ist. Im Förderbetrieb liegt die Folgeplatte im Behälter auf der Flüssigkeit auf und schliesst mit dem Behälter dichtend ab. Die Pumpe fördert die Flüssigkeit durch die Folgeplatte hindurch aus dem Behälter. Wenn die Behälter ausgetauscht werden, kann Luft zwischen die Oberfläche der Flüssigkeit und die Folgeplatte gelangen. Um die Luft von dort zu entfernen, wird zuerst mittels eines am Pumpenausgang montierten Druckaufnehmers der von der Pumpe erzeugte Förderdruck gemessen. Anschliessend wird der gemessene Förderdruck mit einem Soll-Wert verglichen und ein in die Folgeplatte integriertes Entlüftungsventil so oft und so lange geöffnet, bis der Förderdruck mit dem Soll-Wert übereinstimmt. Danach bleibt das Entlüftungsventil geschlossen. Dabei wird davon ausgegangen, dass bei einer luftfreien Flüssigkeit ein ihr spezifischer Förderdruck anliegt, der sich ändert, wenn Luft eingeschlossen ist. Diese Lösung hat allerdings folgenden Nachteil. Falls bei einer Messung festgestellt wird, dass der gemessene Förderdruck dem Soll-Wert entspricht, wird davon ausgegangen, dass von da an nur noch luftfreie Flüssigkeit gefördert wird. Dies ist aber nicht zwangsläufig so. Es kann nämlich auch vorkommen, dass sich zwar nach wie vor Luft in der Flüssigkeit befindet, die verwendete Messprobe aber zufälliger Weise frei von Luft war. In diesem Fall wird bei der oben beschriebenen Lösung später dann trotzdem lufthaltige Flüssigkeit gefördert, ohne dass dies erkannt wurde. Möchte man diesen Nachteil vermeiden, müssten eine Reihe von Messproben gezogen werden und die Flüssigkeit dürfte erst dann zur Spritzgussmaschine gefördert werden, wenn durch mehrere Messungen an verschiedenen Messproben bestätigt wurde, dass die Flüssigkeit keine Luft mehr enthält. Diese Vorgehensweis führt allerdings zu einem hohen Materialausschuss, weil nicht nur die lufthaltigen Messproben, sondern auch die luftfreien Messproben solange entsorgt werden, bis sicher ist, dass keine lufthaltige Messprobe mehr folgen wird. Somit hat die beschriebene Lösung entweder den Nachteil, dass nicht wirklich sichergestellt werden kann, dass luftfreie Flüssigkeit gefördert wird, oder aber, dass relativ viel - auch luftfreie und damit brauchbare - Flüssigkeit entsorgt werden muss, bevor wirklich sichergestellt ist, dass die Flüssigkeit luftfrei ist.

Aus der Druckschrift DE 10 2004 038 801 A1 ist eine Vorrichtung zur Erkennung von Gaseinschlüssen in einem zähflüssigen Medium bekannt. Die Vorrichtung umfasst einen Vorratsbehälter, aus dem das zähflüssige Medium über eine Pumpe und eine Dosiereinrichtung zu einem Messzylinder gefördert wird. Anschliessend wird das Medium im Messzylinder komprimiert und die Kompression mit einer Wegmesseinrichtung erfasst, um damit den im Medium enthaltenen Gasanteil zu ermitteln. Wenn die Kompression des Mediums unter einem vorgegebenen Wert liegt, wird das im Messzylinder befindliche Medium zur Abgabeeinrichtung transportiert. Falls hingegen bei der Kompression des Mediums eine unzulässig hohe Gasmenge im Medium ermittelt wird, wird von der Wegmesseinrichtung ein Störsignal abgegeben, um die Weiterleitung des Mediums an die Abgabeeinrichtung zu verhindern. Der Förderprozess wird unterbrochen.

In der Druckschrift US 2014/0326322 A1 wird ein Messverfahren beschriebenen, wobei dessen Aufgabe darin besteht, eine hochpräzise Volumenmessung durchzuführen. Dabei spielt der Gasanteil im Fluid keine Rolle bezüglich der Frage, ob das Fluid einem nachgeordneten Mischer zugeführt werden soll oder nicht. Auch spielt es keine Rolle, ob das zum Mischer geförderte Fluid gasblasenfrei (brauchbar) oder gasblasenbehaftet (unbrauchbar) ist. Das Fluid wird, unabhängig vom Gasanteil, immer als brauchbar angesehen. Anders als im Stand der Technik wird bei der Erfindung die Möglichkeit geschaffen, zwischen brauchbarem und unbrauchbarem Material unterscheiden zu können und das Material fallweise entsorgen oder weiterzuverwenden zu können.

In der Druckschrift WO 2009/140776 A2 wird eine Vorrichtung zur Entnahme von zwei flüssigen Materialen M1, M2 und aus zwei Vorlagebehältern beschrieben. Die Vorrichtung umfasst zwei Schöpfkolbenpumpen und zwei Dosiereinheiten. Die Menge des geförderten Materials pro Zeiteinheit soll nicht von der Taktung der beiden Schöpfkolbenpumpen abhängen. Zu diesem Zweck werden die beiden Schöpfkolbenpumpen mit einer vorgegebenen unveränderlichen Taktung betrieben. Um das gewünschte Materialverhältnis M1 zu M2 einzustellen, werden die beiden Dosierventileinheiten eingesetzt. Mittels der Dosierventileinheiten ist es möglich, einen bestimmten Teil des von der Schöpfkolbenpumpe geförderten Materials in den Vorlagebehälter zurückzuleiten. Von dieser Möglichkeit wird dann Gebrauch gemacht, wenn sich die Mengen der beiden Materialien M1, M2 in den Vorlagebehältern nicht genau in jenem Verhältnis befinden, das eigentlich zu erwarten ist. Die Aufgabe ist es also eine von der Taktung der Schöpfkolbenpumpen unabhängige Einstellung der Materialverhältnisse M1, M2 zu ermöglichen.

Die Druckschrift DE 20 2011 108 222 U1 beschreibt eine Vorrichtung zum Ausbringen von zwei in Gebinden befindlichen Flüssigkeiten einer Mehrkomponentenrezeptur. Die Aufgabe besteht darin, ein konstantes Mengenverhältnis der geförderten Komponenten zu gewährleisten. Die Vorrichtung umfasst zwei Schöpfkolbenpumpen und zwei Sensoren zur Erfassung der Füllhöhe. Zudem ist eine Steuerung vorgesehen, über die die Fördermengen steuerbar ist, und die dafür sorgt, dass die beiden Komponenten simultan gefördert werden.

### Darstellung der Erfindung

Eine Aufgabe der Erfindung ist es, eine Fördervorrichtung zum Fördern eines viskosen Materials aus einem Behälter und ein Betriebsverfahren anzugeben, bei denen sowohl sichergestellt ist, dass nur blasenfreies Material gefördert als auch der Anteil an zu entsorgendem Material auf ein Minimum reduziert wird.

Die Aufgabe wird durch einen Fördervorrichtung zum Fördern eines viskosen Materials aus einem Behälter mit den in Patentanspruch 1 angegebenen Merkmalen gelöst.

Die erfindungsgemässe Fördervorrichtung zum Fördern eines viskosen Materials aus einem Behälter umfasst eine in den Behälter einsetzbare Folgeplatte und eine Pumpe, mit der das viskose Material durch die Folgeplatte hindurch förderbar ist. Zudem ist eine Messkammer zur Aufnahme einer Messprobe des viskosen Materials vorgesehen. Die Messkammer weist dazu eine verschliessbare Material-Einlassöffnung auf. Von der Messkammer führt eine verschliessbare Entsorgungsleitung weg. Darüber hinaus ist eine von der Messkammer über die Folgeplatte in den Behälter führende verschliessbare Material-Rückführleitung vorgesehen. Die Fördervorrichtung umfasst auch eine Steuerung, die derart ausgebildet und betreibbar ist, dass sie bei mehreren Messproben jeweils deren Kompressibilität ermittelt. Abhängig von der ermittelten Kompressibilität, gibt die Steuerung die Entsorgungsleitung oder die Material-Rückführleitung für die in der Messkammer befindliche Messprobe frei.

Zudem wird ein Verfahren zum Betreiben der oben beschriebenen Fördervorrichtung vorgeschlagen, das folgende Schritte umfasst. Das Material beziehungsweise eine Messprobe des Materials wird in die Messkammer gefördert. Nachdem die Material-Zuführleitung, die Entsorgungsleitung und die Material-Rückführleitung geschlossen sind, wird die Kompressibilität des in die Messkammer befindlichen Materials ermittelt. Mittels einer Steuerung wird, abhängig von der ermittelten Kompressibilität, dafür gesorgt, dass das in der Messkammer befindliche Material über die Entsorgungsleitung entsorgt oder über die Material-Rückführleitung zurück in den Behälter geführt wird.

Die erfindungsgemässe Fördervorrichtung kann zum Fördern eines viskosen Materials verwendet werden.

Vorteilhafte Weiterbildungen der Erfindung ergeben sich aus den in den abhängigen Patentansprüchen angegebenen Merkmalen.

Bei einer Ausführungsform der erfindungsgemässen Fördervorrichtung zum Fördern eines viskosen Materials ist ein Stellglied vorgesehen, mit dem ein bestimmter Druck in der Messkammer einstellbar ist. Zudem ist ein Sensor zur Ermittlung der Änderung des Volumens der Messkammer vorgesehen. Wenn die Messprobe unter Druck gesetzt wird, kann mit Hilfe der erfassten Volumenänderung die Kompressibilität der Messprobe ermittelt werden. Weil Luft um ein Vielfaches kompressibler ist als das viskose Material, kann anhand der Kompressibilität der Messprobe auf einfache Art und Weise mit ausreichend grosser Sicherheit bestimmt werden, ob sich Luft in der Messprobe befindet oder nicht.

Bei einer dazu alternativen Ausführungsform der erfindungsgemässen Fördervorrichtung weist die Pumpe ein Pumpen-Stellglied auf, mit dem ein bestimmtes Volumen in der Messkammer einstellbar ist. Zudem ist ein Sensor zur Ermittlung des Drucks in der Messkammer vorgesehen. Damit kann ebenfalls die Kompressibilität der Messprobe ermittelt werden. Auch dies ist eine Möglichkeit, um bestimmen zu können, ob sich Luft in der Messprobe befindet oder nicht.

Bei einer weiteren Ausführungsform der erfindungsgemässen Fördervorrichtung ist das Stellglied ein Kolben oder eine Membrane. Der Kolben kann zum Beispiel der Kolben der Pumpe oder ein separater Kolben sein.

Bei einer zusätzlichen Ausführungsform der erfindungsgemässen Fördervorrichtung ist ein Sensor vorgesehen, der die Position des Stellglieds erfasst. Sensoren zur Erfassung der Position des Stellglieds sind kostengünstig und liefern zuverlässige und aussagekräftige Messsignale. Mit einem Positionssensor kann die Position des Stellglieds jederzeit und schnell ermittelt werden. Zudem ist ein Positionssensor vielfällig einsetzbar. Mit ihm kann ohne weiteres der Hub eines Kolbens oder einer Membran oder die Winkelposition oder Winkeländerung einer sich drehenden Welle erfasst und zum Beispiel als analoges oder digitales Signal an die Steuerung weitergeleitet werden. Der Positionssensor kann vorteilhafter Weise ausserhalb der Messkammer angeordnet sein. Dadurch bleibt die Kompression der Messprobe vom Sensor unbeeinflusst, was bei einer Druckmessung in der Messkammer mittels eines Drucksensors nicht unbedingt der Fall ist. Der an oder in der Messkammer angeordnete Drucksensor verformt sich und beeinflusst damit selbst das Messkammervolumen.

Bei einer Weiterbildung der erfindungsgemässen Fördervorrichtung nutzt die Steuerung das Signal des Sensors, um die Kompressibilität der in der Messkammer befindlichen Messprobe zu ermitteln.

Bei einer anderen Weiterbildung der erfindungsgemässen Fördervorrichtung ist der Wegsensor als magnetostriktiver Sensor ausgebildet.

Bei einer zusätzlichen Weiterbildung der erfindungsgemässen Fördervorrichtung ist die Messkammer über die Material-Zuführleitung von der Pumpe mit Material versorgbar.

Alternativ dazu kann die Messkammer bei der erfindungsgemässen Fördervorrichtung Teil der Pumpe sein. Dadurch lässt sich die Anzahl der erforderlichen Bauteile reduzieren.

Bei der erfindungsgemässen Fördervorrichtung kann die Pumpe als Kolbenpumpe, Zahnradpumpe, Spindelpumpe oder Exzenter-Schneckenpumpe ausgebildet sein.

Zudem kann die Folgeplatte bei der erfindungsgemässen Fördervorrichtung ein Entlüftungsventil aufweisen. Damit kann vorab zumindest ein Teil jener Luft, die sich in dem Raum zwischen dem zu fördernden Material, der Folgeplatte und der Behälterwand befindet, abgelassen werden.

Darüber hinaus kann bei der erfindungsgemässen Fördervorrichtung vorgesehen sein, dass die Folgeplatte in dem Bereich schräg ausgebildet ist, in dem sich ihre druckwirksame Fläche befindet. Dadurch kann partiell der Druck auf das zu fördernde Material erhöht werden, und es kann dafür gesorgt werden, dass das Material verstärkt in eine bestimmte Richtung strömt, vorzugsweise zur Förderöffnung.

Bei einer Ausführungsform der erfindungsgemässen Fördervorrichtung weist die Material-Zuführleitung ein Ventil auf. Dieses ist vorzugsweise so ausgebildet, dass es von der Steuerung angesteuert werden kann.

Wenn die Messkammer Teil der Pumpe ist, kann das Ventil in der Material-Zuführleitung ein Rückschlagventil sein, das beim Befüllen der Messkammer automatisch öffnet und beim Fördern aus der Messkammer respektive im Messmodus automatisch schliesst.

Die Material-Zuführleitung kann in den Kolben der Pumpe integriert sein.

Bei einer weiteren Ausführungsform der erfindungsgemässen Fördervorrichtung weist die Material-Rückführleitung ein Ventil auf. Dieses ist vorzugsweise so ausgebildet, dass es von der Steuerung angesteuert werden kann.

Bei einer zusätzlichen Ausführungsform der erfindungsgemässen Fördervorrichtung weist die Entsorgungsleitung ein Ventil auf, das vorzugsweise so ausgebildet ist, dass es von der Steuerung angesteuert werden kann.

Zudem wird ein Fördersystem vorgeschlagen, das eine erste und eine zweite, wie oben beschriebene Fördervorrichtung aufweist, wobei die erste Fördervorrichtung und die zweite Fördervorrichtung ausgangsseitig mit einer Mischeinheit verbunden sind. Damit können verschiedene Materialien, wie zum Beispiel eine Komponente A und eine Komponente B, miteinander gemischt und zu einer Verarbeitungsstation, wie zum Beispiel einer Spritzgussmaschine, gefördert werden.

### Kurze Beschreibung der Zeichnungen

Im Folgenden wird die Erfindung mit mehreren Ausführungsbeispielen anhand von zehn Figuren weiter erläutert.
- Figur 1: zeigt ein Fördersystem mit einer möglichen Ausführungsform der erfindungsgemässen Fördervorrichtung in einer dreidimensionalen Ansicht.
- Figur 2: zeigt das Fördersystem mit der erfindungsgemässen Fördervorrichtung in der Seitenansicht.
- Figur 3: zeigt das Fördersystem mit der erfindungsgemässen Fördervorrichtung in der Draufsicht.
- Figur 4: zeigt eine mögliche Ausführungsform einer Messvorrichtung, die Teil der Fördervorrichtung ist, in einer dreidimensionalen Ansicht.
- Figur 5: zeigt die Messvorrichtung im Längsschnitt.
- Figur 6: zeigt eine mögliche Ausführungsform der Pumpe der Fördervorrichtung im Längsschnitt.
- Figur 7a: bis d zeigt anhand von vier Blockschaltbildern verschiedene Betriebszustände der Fördervorrichtung.
- Figur 8a: bis d zeigt anhand von vier Blockschaltbildern verschiedene Betriebszustände einer weiteren möglichen Ausführungsform der Fördervorrichtung.
- Figur 9a: bis d zeigt anhand von vier Blockschaltbildern verschiedene Betriebszustände einer anderen möglichen Ausführungsform der Fördervorrichtung.
- Figur 10a: bis c zeigt anhand von drei Blockschaltbildern verschiedene Betriebszustände einer zusätzlichen möglichen Ausführungsform der Fördervorrichtung.

### Wege zur Ausführung der Erfindung

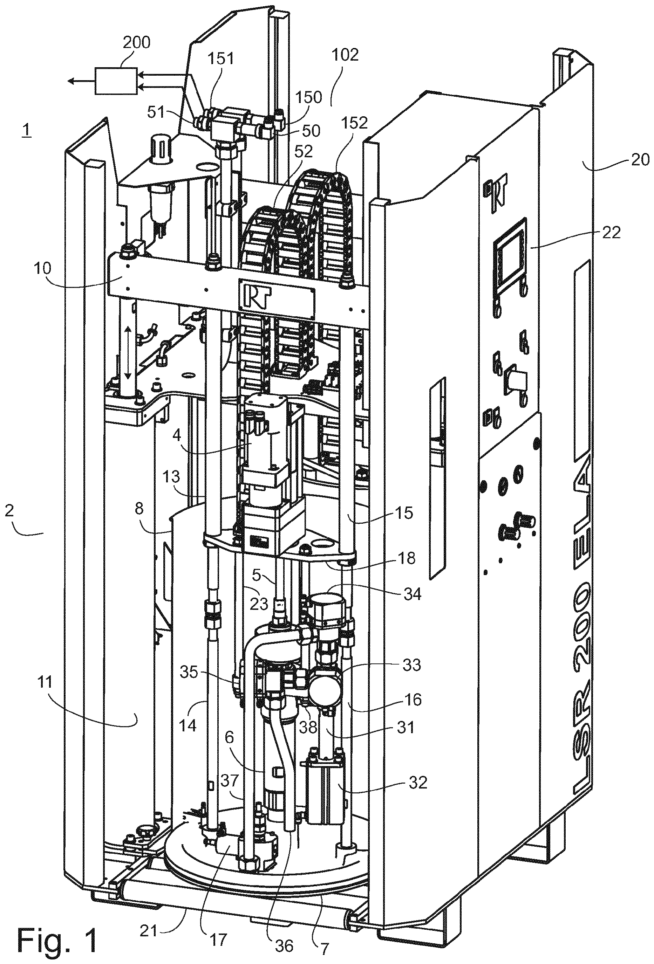

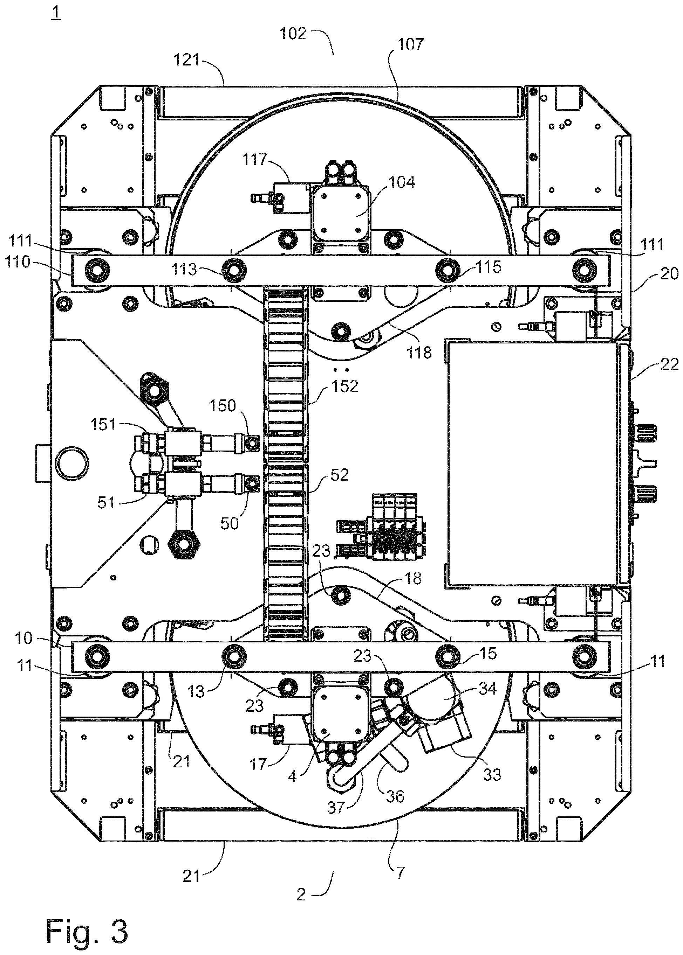

Im Folgenden wird der Aufbau einer ersten möglichen Ausführungsform eines Fördersystems 1 zum Fördern von viskosem Material weiter erläutert. Das in den Figuren 1, 2 und 3 dargestellte Fördersystem 1 umfasst eine erste Fördervorrichtung 2, mit der zum Beispiel eine Komponente A förderbar ist, und eine zweite Fördervorrichtung 102, mit der zum Beispiel eine Komponente B gefördert werden kann. Grundsätzlich können die beiden Fördervorrichtungen 2 und 102 gleich aufgebaut sein. Dies ist aber nicht zwingend notwendig. Im Folgenden wird davon ausgegangen, dass die beiden Fördervorrichtungen 2 und 102 baugleich sind. Dabei ist das Bezugszeichen des Bauteils der zweiten Fördervorrichtung 102, gegenüber dem Bezugszeichen desjenigen Bauteils der ersten Fördervorrichtung 2, mit dem es baugleich ist, um 100 erhöht.

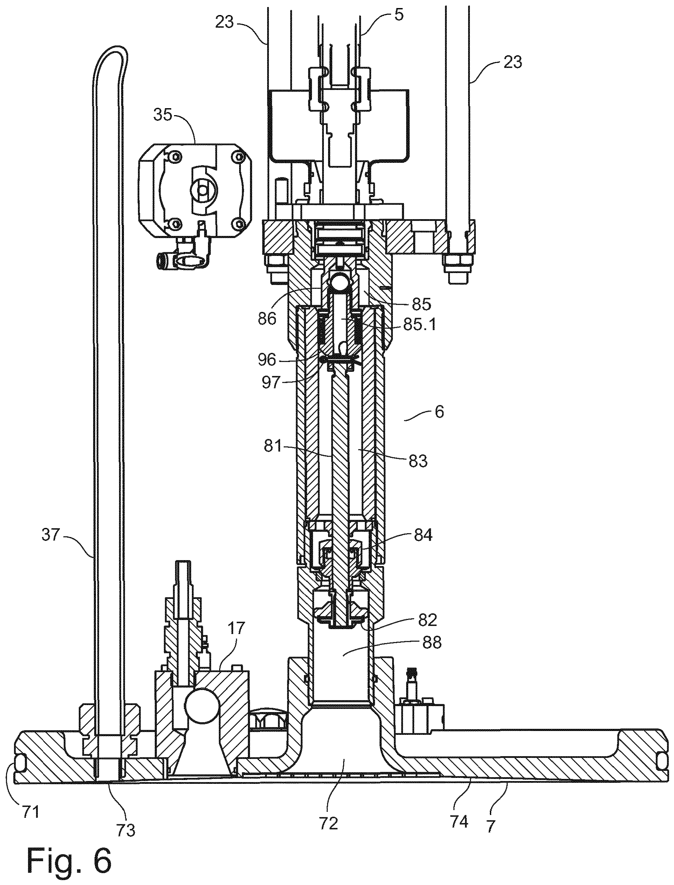

Die Fördervorrichtung 2 umfasst eine Pumpe 6, die über einen Antrieb 4 und eine Antriebsstange 5 angetrieben wird. Wenn die Pumpe 6, wie in Figur 6 gezeigt, als Schöpfkolbenpumpe ausgebildet ist, ist der Antrieb 4 so gestaltet, dass er die Antriebsstange 5 veranlasst eine Hubbewegung auszuführen, die auf die Schöpfkolbenpumpe übertragen wird. Die Pumpe 6 kann statt der Schöpfkolbenpumpe auch eine Kolbenpumpe ohne Schöpfkolben sein. Der Schöpfkolben hat bei hoch viskosen Materialien den Vorteil, dass das viskose Material direkt zum Pumpeneingang geschöpft wird. Damit kann das Ansaugverhalten der Pumpe verbessert werden. Wenn die Pumpe 6 hingegen als Zahnradpumpe, Spindelpumpe oder als Exzenter-Schneckenpumpe ausgebildet ist, ist der Antrieb so gestaltet, dass er die Antriebsstange 5 veranlasst eine Rotationsbewegung auszuführen, die auf die Zahnradpumpe, die Spindelpumpe beziehungsweise Exzenter-Schneckenpumpe übertragen wird.

Im vorliegenden Ausführungsbeispiel wird davon ausgegangen, dass die Pumpe 6 eine Schöpfkolbenpumpe ist. Figur 6 zeigt anhand eines Längsschnitts beispielhaft wie eine solche Schöpfkolbenpumpe ausgebildet sein kann. Unterhalb der Pumpe 6 befindet sich eine Folgeplatte 7, die in einen Behälter mit dem zu fördernden Material eingeführt werden kann. Die Folgeplatte 7 ist vorzugsweise mit einer ringförmigen Dichtung 71 bestückt, damit die Folgeplatte 7 an der Behälterwand dichtend anliegen kann. Die Dichtung 71 sorgt dafür, dass zwischen der Folgeplatte 7 und der Behälterwand kein Material austritt. Die Folgeplatte 7 weist eine druckwirksame Fläche 74 auf, die vorteilhafter Weise zumindest partiell schräg ausgebildet ist. Dadurch kann partiell der Druck auf das zu fördernde Material erhöht und dafür gesorgt werden, dass das Material verstärkt zur Förderöffnung 72 der Folgeplatte 7 strömt. Durch die Förderöffnung 72 kann die Pumpe 6 das viskose Material aus dem Behälter herausfördern. Die Schöpfkolbenpumpe weist dazu einen Schöpfkolben 81 auf, wobei sich an dessen unteren Ende ein Schöpfkolbenteller 82 befindet. Um Material aus dem Behälter heraus zu fördern, wird der Schöpfkolben 81 nach unten bewegt, sodass der Schöpfkolbenteller 82 in das Material eintaucht und Material aufnimmt. In der Aufwärtsbewegung des Schöpfkolbens 81 nimmt der Schöpfkolbenteller 82 das Material durch die Förderöffnung 72 hindurch mit ins Pumpeninnere. Von dort gelangt es über ein erstes Rückschlagventil 84 nach oben in eine untere Pumpenkammer 83. Bei der nächsten Abwärtsbewegung der Antriebsstange 5 und des mit ihr verbundenen Schöpfkolbens 81 gelangt das Material über ein zweites Rückschlagventil 86 in eine obere Pumpenkammer 85. Mit der darauf folgenden Aufwärtsbewegung des Schöpfkolbens 81 wird das Material aus der Pumpe 6 heraus in eine Material-Zuführleitung 38 transportiert. Damit wird mit jedem Aufwärtshub des Schöpfkolbens 81 Material in die Material-Zuführleitung 38 transportiert. Das erste Rückschlagventil 84 kann als Sitzventil und das zweite Rückschlagventil 86 kann als Kugelventil ausgebildet sein.

Der Antrieb 4 ist auf einer Motorhalterung 18 befestigt. Diese wiederum ist mittels einer ersten und einer zweiten Stange 13, 15 an einem Joch 10 befestigt. Die beiden Stangen 13 und 15 können als Rohre ausgebildet sein und dienen auch als Führung für zwei Hubstangen 14 und 16. An den beiden Hubstangen 14 und 16 ist die Folgeplatte 7 befestigt. Die Pumpe 6 kann mittels weiterer Stangen 23 an der Motorhalterung 18 befestigt sein.

Das Joch 10 wird von zwei Hubzylindern 11 getragen. Die Hubzylinder 11, das Joch 10 und die Stangen 13 bis 16 bilden einen Pumpenheber, der dazu dient, den Antrieb 4 mitsamt der Pumpe 6 und die Folgeplatte 7 heben und senken zu können.

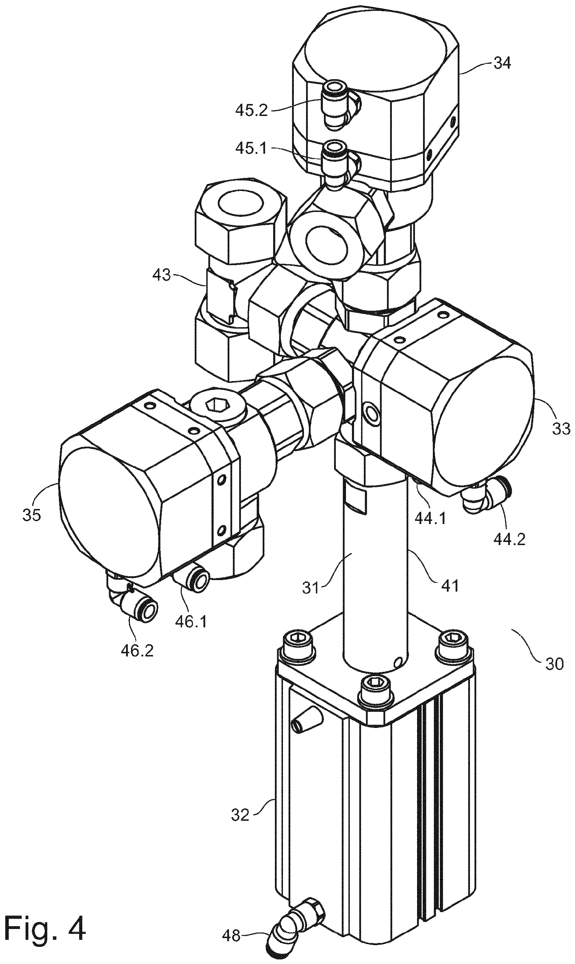

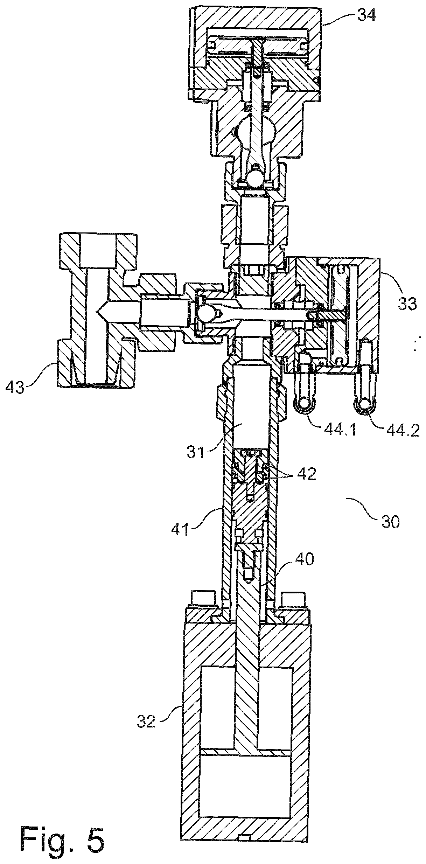

Die in den Figuren 4 und 5 dargestellte Messvorrichtung 30 ist eine mögliche Ausführungsform und kann Teil der Fördervorrichtung 2 sein. In Figur 4 ist die Messvorrichtung 30 in einer dreidimensionalen Ansicht und in Figur 5 in der Seitenansicht im Schnitt dargestellt. Die Messvorrichtung 30 umfasst in einem Messkammergehäuse 41 eine Messkammer 31, wobei das Volumen der Messkammer 31 mit Hilfe eines Kolbens 40 verändert werden kann. Die Position des Kolbens 40 kann mit einem pneumatischen Zylinder 32 eingestellt werden. Je weiter der Kolben 40 aus dem Zylinder 32 heraus und damit in die Messkammer 31 hineinragt, desto kleiner wird das Volumen in der Messkammer 31. Am oberen Ende des Kolbens 40 befindet sich eine Kolbendichtung 42, um die Messkammer 31 nach unten zum Zylinder 32 hin abzudichten. Um den Zylinder 32 mit Druckluft DL beaufschlagen zu können, ist am Zylinder 32 ein entsprechender Steueranschluss 48 vorgesehen. Über den Steueranschluss 48 kann der Kolben 40 im Zylinder 32 mit Druckluft DL beaufschlagt und bewegt werden. Das Messkammergehäuse 41 kann, wie in den Figuren 4 und 5 gezeigt, rohrförmig ausgebildet sein. Am oberen Ende des Messkammergehäuses 41 befindet sich eine Öffnung, über die das zu untersuchende Material, also die Messprobe, in die Messkammer 31 gelangen kann, und über die die Messprobe auch wieder aus der Messkammer 31 herausbefördert werden kann.

Über ein zum Beispiel als Nadelventil ausgebildetes Einlassventil 33 kann das zu untersuchende Material über eine Material-Zuführleitung 38 (siehe Figuren 1 bis 3) in die Messkammer 31 gefördert werden. Die Material-Zuführleitung 38 wird im Folgenden auch kurzum als Zuführleitung bezeichnet. Sie kann über ein T-Stück 43 mit dem Einlassventil 33 verbunden sein. Wird die Messeinheit 30 nicht benötigt, kann das geförderte Material über das T-Stück 43 woanders hin geleitet werden. Das Einlassventil 33 kann als pneumatisch betriebenes Ventil mit zwei Steueranschlüssen 44.1 und 44.2 ausgebildet sein. Wird der Steueranschluss 44.1 mit Druckluft beaufschlagt, sorgt der Kolben im Ventil 33 dafür, dass der Materialeinlass des Ventils geöffnet wird, sodass die Messprobe in die Messkammer 31 gelangt. Wird stattdessen der Steueranschluss 44.2 mit Druckluft beaufschlagt, sorgt der Kolben im Einlassventil 33 dafür, dass der Materialeinlass des Ventils geschlossen wird.

Über ein Auslassventil 34, das auch als Rückführventil bezeichnet wird, kann das zu untersuchende Material/Messprobe über eine Material-Rückführleitung 37 (siehe Figuren 1 bis 3) aus der Messkammer 31 heraus und zurück in den Behälter mit dem zu fördernden Material gefördert werden. Die Material-Rückführleitung 37 wird im Folgenden auch kurzum als Rückführleitung bezeichnet. Das Auslassventil 34 kann als pneumatisch betriebenes Ventil mit zwei Steueranschlüssen 45.1 und 45.2 ausgebildet sein. Wird der Steueranschluss 45.1 mit Druckluft beaufschlagt, sorgt der Kolben im Auslassventil 34 dafür, dass der Materialauslass des Ventils geöffnet wird. Wird stattdessen der Steueranschluss 45.2 mit Druckluft beaufschlagt, sorgt der Kolben im Auslassventil 34 dafür, dass der Materialauslass des Ventils geschlossen wird, sodass die Rückführleitung 37 geschlossen ist.

Über ein weiteres Auslassventil 35 kann das zu untersuchende Material über eine Material-Entsorgungsleitung 36 (siehe Figuren 1 bis 3) aus der Messkammer 31 heraus und zum Beispiel in einen nicht gezeigten Abfallbehälter fördert werden. Die Material-Entsorgungsleitung 37 wird im Folgenden auch kurzum als Entsorgungsleitung bezeichnet. Das Auslassventil 35 kann als pneumatisch betriebenes Ventil mit zwei Steueranschlüssen 46.1 und 46.2 ausgebildet sein. Wird der Steueranschluss 46.1 mit Druckluft beaufschlagt, sorgt der Kolben im Auslassventil 35 dafür, dass der Materialauslass des Ventils geöffnet wird. Wird stattdessen der Steueranschluss 46.2 mit Druckluft beaufschlagt, sorgt der Kolben im Auslassventil 35 dafür, dass der Materialauslass des Ventils geschlossen wird und kein Material in die Entsorgungsleitung 36 gelangt.

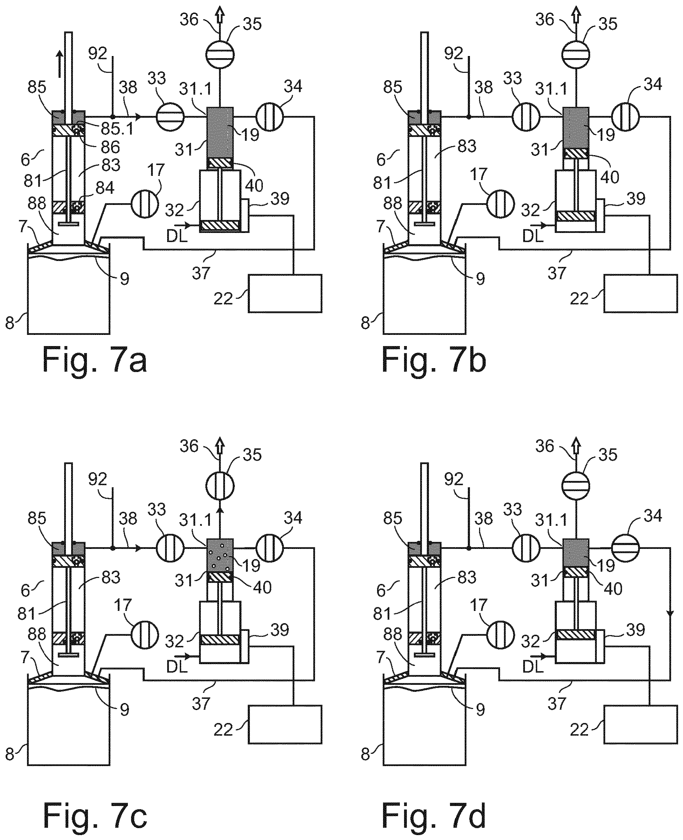

In den Figuren 7a bis 7d sind anhand von vier Blockschaltbildern vier verschiedene Betriebszustände der Fördervorrichtung 2 dargestellt. Die gezeigten Betriebszustände gelten sinngemäss auch für die Fördervorrichtung 102, falls sie vorhanden ist.

Die Fördervorrichtung 2 kann im Fördermodus oder im Entlüftungsmodus betrieben werden.

Im Fördermodus fördert die Fördervorrichtung 2 das viskose Material mit Hilfe der Pumpe 4 aus dem Behälter 8 heraus.

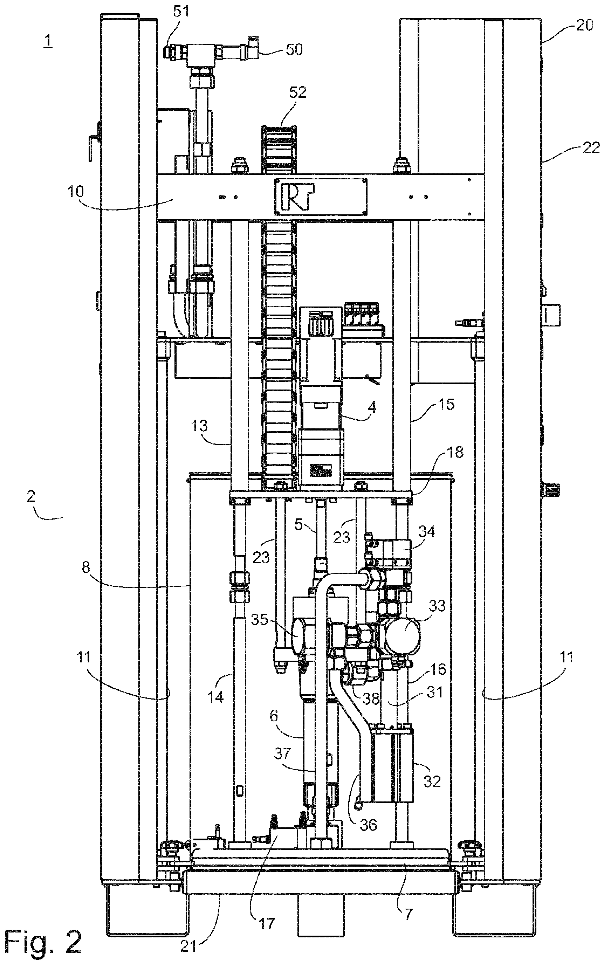

Bei der in den Figuren 1 und 2 dargestellten Ausführungsform fördert die Fördervorrichtung 2 die Komponente A zum Auslass 51 und die Fördervorrichtung 102 die Komponente B zum Auslass 151. Von dort können die beiden Komponenten A und B einer Mischeinheit 200 und/oder einer Verarbeitungsstation zugeführt werden. An den Auslässen 51 und 151 können Druckaufnehmer 50 und 150 vorgesehen sein.

Wenn der Behälter in der Fördervorrichtung 2 leer gepumpt ist, wird der Fördermodus unterbrochen. Mit Hilfe des Pumpenhebers wird dann die Pumpe 4 mitsamt der Folgeplatte 7 soweit angehoben, bis sich die Folgeplatte 7 oberhalb des Behälters befindet. Nun wird der leere Behälter aus der Fördervorrichtung 2 herausgezogen und ein voller Behälter in die Fördervorrichtung 2 hineingeschoben und unter der Folgeplatte 7 positioniert, wobei Transportrollen 21 das Herausziehen und Hineinschieben der Behälter erleichtern.

Anschliessend wird mit Hilfe des Pumpenhebers der Pumpenantrieb 4 und die Pumpe 6 mitsamt der Folgeplatte 7 soweit abgesenkt, bis die Folgeplatte 7 im Behälter auf dem zu fördernden Material 9 aufliegt. Hierbei kann es vorkommen, dass Luft zwischen der Oberfläche des zu fördernden Materials 9, der Behälterwand und der Folgeplatte 7 eingeschlossen wird.

Im Folgenden wird der Entlüftungsmodus weiter beschrieben.

Um die eingeschlossene Luft von dort zu entfernen, kann man in einer ersten Entlüftungsphase die Folgeplatte 7 zuerst für eine bestimmte Zeitdauer auf dem zu fördernden Material 9 ruhen lassen, um dem Material Zeit zu geben, sich zu verteilen. Die Zeitdauer (Ruhezeit) kann zum Beispiel auf die Viskosität des Materials abgestimmt sein. Anschliessend wird das Entlüftungsventil 17 an der Folgeplatte 7 geöffnet, sodass die unter der Folgeplatte 7 befindliche Luft entweichen kann. Dann wird das Entlüftungsventil 17 wieder geschlossen. Dieses Vorgehen ist von Vorteil, aber nicht zwingend notwendig.

In einer zweiten Entlüftungsphase wird die restliche Luft aus dem Behälter entfernt. Dabei kann mit der Steuerung 22 zwischen brauchbarem (luftfreien) und unbrauchbarem (luftbehafteten) Material unterschieden werden. Dazu wird das Einlassventil 33 geöffnet, das Auslassventil 34 und das Ablassventil 35 sind geschlossen. Für den Entlüftungsmodus ist es von Vorteil, wenn die Material-Transportleitung 92 geschlossen ist. Das Material 9 beziehungsweise die Messprobe 19 wird über die Material-Zuführleitung 38 aus dem Behälter 8 heraus und in die Messkammer 31 hinein transportiert (Figur 7a). Dies geschieht dadurch, dass der Zylinder 32 den Kolben 40 aus der der Messkammer 31 herauszieht. Somit wird das Volumen in der Messkammer 31 vergrössert und ein Unterdruck in der Messkammer 31 erzeugt, der das Material in die Messkammer 31 saugt. Alternativ dazu oder als Unterstützung dazu kann das Material auch mittels der Pumpe 6 in die Messkammer 31 gepumpt werden.

Anschliessend wird die Material-Zuführleitung 38 geschlossen, indem das Einlassventil 33 geschlossen wird (Figur 7b). Die beiden Auslassventile 34 und 35 bleiben geschlossen. Der Zylinder 32 wird nun mit einem definierten Druck beaufschlagt, so dass über den Kolben 40 auch in der Messkammer 31 ein definierter Druck p herrscht. In einem nächsten Schritt wird der Weg x gemessen, den der Kolben 40 zurückgelegt hat. Der zurückgelegte Weg x ist dabei ein Mass für die Kompressibilität des in der Messkammer 31 befindlichen Materials also der Messprobe 19. Wenn sich Luft in der Messkammer 31 und damit im Material befindet, legt der Kolben 40 einen weiteren Weg zurück als wenn das Material blasenfrei ist. Der Grund hierfür ist, dass das Material selbst kaum kompressibel ist, die Luft hingegen ist im Vergleich zum Material stark kompressibel. Die Steuerung 22 vergleicht den gemessenen zurückgelegten Weg x des Kolbens 40 mit einem Referenzwert xref.

Falls die Steuerung 22 feststellt, dass der gemessene zurückgelegte Weg x grösser als der Referenzwert xref ist, wird das Material als nicht blasenfrei klassifiziert (Figur 7c). In diesem Fall wird das Auslassventil 35 geöffnet, die beiden anderen Ventile 33 und 34 bleiben geschlossen. Die Messprobe 19 (Material und Luft) wird mit Hilfe des Kolbens 40 aus der Messkammer 31 herausgedrückt und über die Entsorgungsleitung 36 zum Beispiel in einen Abfallbehälter entsorgt.

Falls das in der Messkammer 31 befindliche Material von der Steuerung 22 als blasenfrei und damit brauchbar klassifiziert wurde (Figur 7d), wird das Auslassventil 34 geöffnet, die beiden anderen Ventile 33 und 35 bleiben geschlossen. Das Material (Messprobe 19) wird mit Hilfe des Kolbens 40 aus der Messkammer 31 herausgedrückt und über die Material-Rückführleitung 37 wieder in den Behälter 8 zurücktransportiert.

Um sicher zu gehen, dass sich keine Restluft mehr im Behälter 8 befindet, kann der oben beschriebene Vorgang noch einmal oder mehrmals wiederholt werden. Dazu wird das Auslassventil 34 wieder geschlossen und das Einlassventil 33 geöffnet. Das Ablassventil 35 bleibt geschlossen (Figur 7a). Anschliessend wird eine weitere Messprobe (neues Material) aus dem Behälter 8 heraus und in die Messkammer 31 hinein transportiert. Nun können die oben erwähnten Schritte erneut durchgeführt werden. Wenn die Steuerung 22 auch nach mehrmaligem Durchführen der oben genannten Schritte bei keiner Kompressionsmessung und also bei keiner Messprobe mehr Material als blasenbehaftet klassifiziert, kann davon ausgegangen werden, dass sich keine Luft mehr zwischen der Folgeplatte 7, der Oberfläche des zu fördernden Materials 9 und der Behälterwand befindet. Um dies sicherzustellen, kann zum Beispiel folgendes vorgesehen sein. Erst wenn die Steuerung 22 bei sechs aufeinanderfolgenden Messungen und damit bei sechs aufeinanderfolgenden Messproben 19 das geprüfte Material als blasenfrei klassifiziert hat, wird das im Behälter 8 befindliche Material 9 als blasenfrei erachtet.

Um die Wahrscheinlichkeit für eine fälschliche Freigabe des Materials 8 zur Förderung noch weiter zu reduzieren, kann auch folgendes vorgesehen sein. Erst dann, wenn die Steuerung 22 bei acht aufeinander folgenden Messungen und damit bei acht aufeinander folgenden Messproben 19 das geprüfte Material als blasenfrei klassifiziert hat, wird das gesamte Material als blasenfrei erachtet.

Je höher also die Anzahl der als blasenfrei erkannten Messproben 19 ist, desto geringer ist die Wahrscheinlichkeit für eine fälschliche Förderfreigabe des Materials 9.

Wenn die Steuerung 22 nach einer Reihe von untersuchten Messproben 19 das Material 9 im Behälter 8 als brauchbar deklariert hat, kann sie veranlassen, dass die Fördervorrichtung 2 vom Entlüftungsmodus zurück in den Fördermodus wechselt.

Alternativ dazu kann die Steuerung 22 zum Beispiel so ausgebildet sein, dass sie folgendes durchführen kann. Es wird in einem ersten Prüfzyklus eine erste Messprobe gezogen und dahingehend ausgewertet, ob sich darin Luft befindet. Falls das Material der Messprobe als blasenfrei klassifiziert worden ist, wird es über die Material-Rückführleitung 37 wieder in den Behälter 8 zurück transportiert. Falls das geprüfte Material als nicht blasenfrei klassifiziert worden ist, wird es über die Material-Entsorgungsleitung 36 entsorgt. Anschliessend wird in einem zweiten Prüfzyklus eine zweite Messprobe gezogen, wobei mit ihr auf die gleiche Art und Weise umgegangen wird wie mit der ersten Messprobe. Insgesamt können so n aufeinanderfolgende Prüfzyklen mit n Messproben durchgeführt werden, wobei jeder der n Prüfzyklen sinngemäss auf die gleiche Weise abläuft wie der erste Prüfzyklus. Nach einem Fasswechsel können so zum Beispiel n = 8 Prüfzyklen durchlaufen und damit n = 8 Messproben gezogen werden; nach dem achten Prüfzyklus schaltet die Steuerung 22 vom Entlüftungsmodus in den Fördermodus um. Die Anzahl n der aufeinanderfolgenden Prüfzyklen kann aber auch zum Beispiel n = 5, 6, 9 oder 10 sein. Je mehr Prüfzyklen durchlaufen werden, desto geringer ist die Wahrscheinlichkeit, dass die Steuerung 22 das Material 9 fälschlicherweise zur Förderung freigibt, das heisst fälschlicherweise vom Entlüftungsmodus in den Fördermodus umschaltet.

Es ist auch eine Mischform zwischen den beiden oben beschriebenen Abläufen möglich. So kann die Steuerung 22 zum Beispiel wie folgt ausgebildet und betreibbar sein. Es werden mindestens sieben Prüfzyklen durchlaufen, wobei nur dann, wenn bei den, zeitlich gesehen, letzten drei Prüfzyklen das geprüfte Material jeweils als blasenfrei klassifiziert wurde, vom Entlüftungsmodus in den Fördermodus umschaltet wird. Andernfalls sorgt die Steuerung 22 dafür, dass noch ein oder mehrere weitere Prüfzyklen durchgeführt werden.

Von der Art des Materials 9 und den Bedürfnissen bezüglich einer fehlerfreien Entscheidung, ob und wann vom Entlüftungsmodus in den Fördermodus umgeschaltet werden soll, kann es abhängen wieviel Material die Steuerung 22 im Entlüftungsmodus prüfen soll. Wenn die Steuerung zum Beispiel zwischen 2 bis 4 Liter Material prüfen soll, werden etwa 40 bis 80 Prüfzyklen durchlaufen. Der Maschinenbediener kann die Anzahl der Prüfzyklen selbst einstellen. Er kann zum Beispiel auch bestimmen, dass die Steuerung 22 vom Entlüftungsmodus in den Fördermodus umgeschaltet, wenn in den Messproben 19 nach zum Beispiel zehn Prüfzyklen keine Luftblasen mehr erkannt werden.

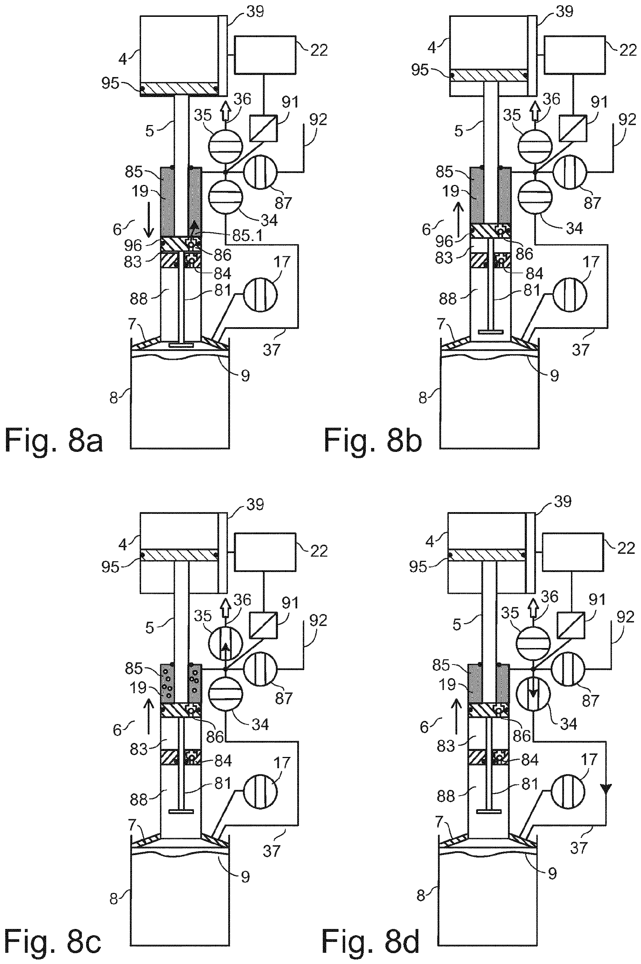

In den Figuren 8a bis 8d sind anhand von vier Blockschaltbildern vier verschiedene Betriebszustände einer weiteren möglichen Ausführungsform der Fördervorrichtung 2 dargestellt. Die zweite Ausführungsform der Fördervorrichtung 2 unterscheidet sich von den oben beschriebenen Ausführungsformen der Fördervorrichtung dadurch, dass die Messkammer 31 nicht in einem separaten Messkammergehäuse 41 untergebracht ist, sondern Teil der Pumpe 6 ist. Die Pumpenkammer 85 dient hier zugleich auch als Messkammer, mit der die Kompressibilität des zu fördernden Materials beziehungsweise der Messprobe 19 bestimmt wird.

Die Kolbenpumpe gemäss Figur 8 ist doppelwirkend, das heisst die Kolbenpumpe fördert am Pumpenausgang sowohl beim Aufwärtshub als auch beim Abwärtshub.

Beim Abwärtshub wird die Messkammer zur Ermittlung der Kompressibilität der Messprobe 19 durch die untere Kammer 83 und die obere Kammer 85 gebildet (Figur 8a). Beim Aufwärtshub wird die Messkammer durch die obere Kammer 85 gebildet (Figur 8b).

Grundsätzlich funktioniert die in den Figuren 8a bis 8d beschriebene Ausführungsform der Fördervorrichtung 2 auf die gleiche Art und Weise wie die in den Figuren 7a bis 7d beschriebene Ausführungsform. Auch hier kann die Fördervorrichtung 2 im Fördermodus oder im Entlüftungsmodus betrieben werden. Im Fördermodus fördert die Fördervorrichtung 2 das viskose Material mit Hilfe der Pumpe 6 aus dem Behälter 8 heraus.

Der Entlüftungsmodus kann, wie bereits oben beschrieben, eine erste und eine zweite Entlüftungsphase umfassen. Um Wiederholungen zu vermeiden, wird bezüglich der Erläuterung der ersten Entlüftungsphase auf die obige Beschreibung verwiesen.

In der zweiten Entlüftungsphase wird die restliche Luft aus dem Behälter 8 entfernt.

Dazu kann zu Beginn der zweiten Entlüftungsphase vorgesehen sein, dass die Steuerung 22 das Rückführventil 34, das Ablassventil 35 und das Förderventil 87 schliesst. Anschliessend wird der Kolben 96 mit Hilfe der Antriebsstange 5 nach oben bewegt, sodass die Pumpenkammer/Messkammer 85 unter einen definierten Druck gesetzt wird. Mit einem Weg-Sensor 39 wird der Weg gemessen, den der Kolben 95 dabei zurückgelegt hat. Die Pumpenkammer dient zugleich auch als Messkammer 85 und der gemessene Weg ist ein Mass für die Volumenänderung in der Messkammer 85. Falls die Messung ergibt, dass sich noch brauchbares Material in der Messkammer 85 befindet (in der Regel ist dies Material aus der vorherigen Charge), veranlasst die Steuerung 22, dass das Rückführventil 34 geöffnet wird. Andernfalls veranlasst sie, dass das Ablassventil 35 geöffnet wird und das Rückführventil 34 geschlossen bleibt. Nun wird der Kolben 96 mit Hilfe der Antriebsstange 5 ganz nach oben bewegt, sodass das in der Messkammer 85 befindliche Material aus der Messkammer über dasjenige Ventil 34 bzw. 35 heraustransportiert wird, das von der Steuerung 22 geöffnet wurde. Gleichzeitig wird mit der Aufwärtsbewegung der Antriebsstange 5 auch der Schöpfkolben 81 nach oben bewegt. Dabei fördert der Schöpfkolben 81 Material 9 aus dem Behälter 8 heraus und durch die Kammer 88 über das Rückschlagventil 84 in die untere Pumpenkammer 83.

Nachdem der Kolben 96 seine obere Endlage erreicht hat und damit das Material aus der Pumpenkammer 85 herausbefördert ist, wird das vorher geöffnete Ventil 34 bzw. 35 wieder geschlossen. Jetzt kann eine weitere Kompressionsmessung durchgeführt werden. Dazu werden die Antriebstange 5 und der Kolben 96 nach unten gedrückt. Da nun sämtliche auslassseitigen Ventile 34, 35 und 87 am Auslass der Pumpe 6 geschlossen sind, wird durch die Abwärtsbewegung der Antriebstange 5 das Gesamtvolumen (Volumen der Kammer 83 plus Volumen der Kammer 85) reduziert und die Messprobe 19 komprimiert. Jetzt kann auf die bereits oben erwähnte Art und Weise die Kompressibilität der Messprobe 19 bestimmt werden. Sobald die Kompressionsmessung abgeschlossen ist, kann nun, abhängig vom Ergebnis der Kompressionsmessung und der Materialklassifizierung (wie vorhin beschrieben) das Rückführventil 34 oder das Ablassventil 35 geöffnet werden. Durch den nun folgenden Abwärtshub des Kolbens 96 wird die untere Kammer 83 geleert. Der eine Teil des Materials wird aus der unteren Pumpenkammer 83 heraus, über das Rückschlagventil 86 in die obere Pumpenkammer 85 und von dort aus der Pumpe 6 herausgefördert. Der andere Teil des Materials wird ebenfalls aus der unteren Kammer 83 heraus befördert, verbleibt aber in der oberen Kammer 85.

Anschliessend wird das vorher geöffnete Ventil 34 beziehungsweise 35 wieder geschlossen. Figur 8a zeigt jenen Betriebszustand der Fördervorrichtung, bei dem sich der Kolben 96 in seiner unteren Endlage befindet und das Ventil 34 beziehungsweise 35 wieder geschlossen ist.

Nachdem der Kolben 96 seine untere Endlage erreicht hat, wird in einem nächsten Schritt bei geschlossenem Rückschlagventil 86 die Antriebsstange 5 mit dem Kolben 96 wieder nach oben gezogen (Figur 8b). Dabei wird die Messprobe 19 nun in der oberen Pumpenkammer/Messkammer 85 mit einem definierten Druck p beaufschlagt.

In einem weiteren Schritt wird mit dem Wegsensor 39 der Weg x gemessen, den die Antriebsstange 5 zurückgelegt hat. Der Wegsensor 39 kann beispielsweise den Hub des mit der Antriebsstange 5 verbundenen Kolbens 95 erfassen. Der zurückgelegte Weg x ist dabei ein Mass für die Volumenänderung beziehungsweise die Kompressibilität der in der Pumpenkammer/Messkammer 85 befindlichen Messprobe 19. Wenn sich Luft in der Pumpenkammer/Messkammer 85 und damit im Material befindet, legt die Antriebsstange 5 einen weiteren Weg zurück als wenn das Material blasenfrei ist. Die Steuerung 22 vergleicht nun den gemessenen zurückgelegten Weg x des der Antriebsstange 5 mit einem Referenzwert xref. Die Auswertung der Messergebnisse und die Klassifikationen der Messproben 19 erfolgen nun sinngemäss wie bereits oben beschrieben.

Wenn der Luftmotor 4 mit einem definierten Luftdruck betrieben wird, kann die im Luftmotor 4 entstehende Reibung zu einem Fehler bei der Ermittlung der Kompressibilität führen. Um diesen Effekt zu kompensieren und um die Kompressibilität der Messprobe 19 noch genauer bestimmen zu können, kann die Fördervorrichtung, wie in Figur 8 gezeigt, zusätzlich auch einen Drucksensor 91 aufweisen. Der Drucksensor 91 dient dazu den effektiven Druck in der Messkammer 85 zu messen.

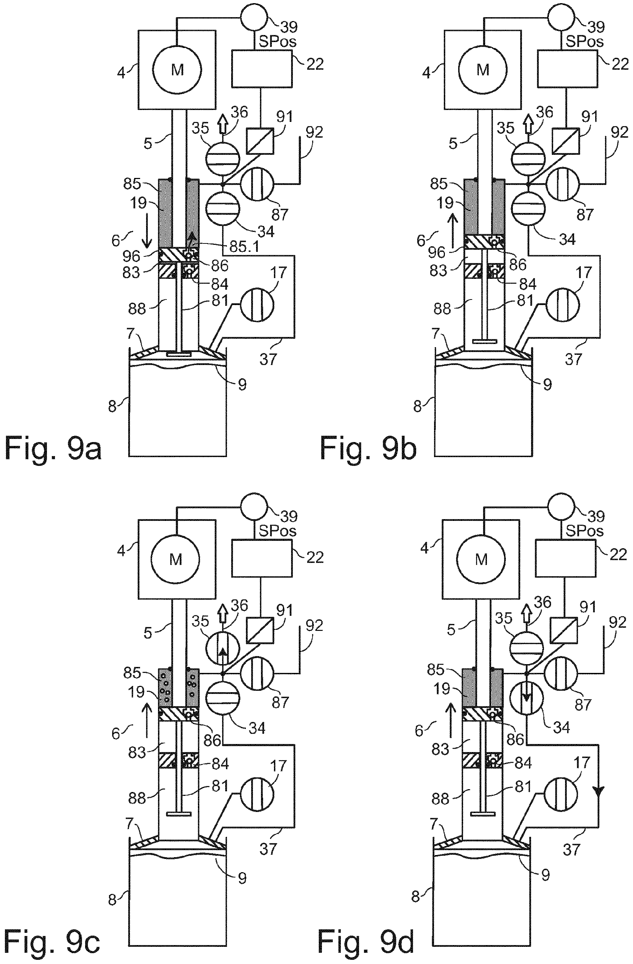

In den Figuren 9a bis 9d sind anhand von vier Blockschaltbildern vier verschiedene Betriebszustände einer weiteren möglichen Ausführungsform der Fördervorrichtung 2 dargestellt. Die weitere Ausführungsform der Fördervorrichtung 2 unterscheidet sich von der oben beschriebenen Ausführungsform gemäss Fig. 8 dadurch, dass der Pumpenantrieb 4 nicht als Luftmotor, sondern als Elektromotor und vorzugsweise als Servomotor ausgebildet ist.

Der Servomotor 4 liefert über einen integrierten Inkrementalgeber ein Positionssignal, über das sich die Drehlage der Antriebswelle des Servomotors, die Position der Stange 5 beziehungsweise die Position des Kolbens 96 bestimmen lässt. Das Positionssignal wird an die Steuerung 22 geleitet.

Grundsätzlich funktioniert die in den Figuren 9a bis 9d beschriebene Ausführungsform der Fördervorrichtung 2 auf die gleiche Art und Weise wie die in den Figuren 8a bis 8d beschriebene Ausführungsform. Auch hier kann die Fördervorrichtung 2 im Fördermodus oder im Entlüftungsmodus betrieben werden. Im Fördermodus fördert die Fördervorrichtung 2 das viskose Material mit Hilfe der Pumpe 6 aus dem Behälter 8 heraus.

Der Entlüftungsmodus kann, wie bereits oben beschrieben, eine erste und eine zweite Entlüftungsphase umfassen. Um Wiederholungen zu vermeiden, wird bezüglich der Erläuterung der Entlüftungsphasen auf die obige Beschreibung verwiesen und insbesondere auf die betreffend die Ausführungsform gemäss Figur 8. Statt den zurückgelegten Weg x der Antriebsstange 5 zu erfassen, kann bei der Ausführungsform gemäss Figur 9 auch die Drehlage der Antriebswelle des Servomotors erfasst werden.

Die Kalibrierung kann dadurch erfolgen, dass der Servomotor bei leeren Kammern 83 und 85 einmal in die eine Endlage fährt und die Steuerung 22 dann das anliegende Positionssignal speichert. Anschliessend fährt der Servomotor in die andere Endlage und die Steuerung 22 speichert dann das nun anliegende Positionssignal.

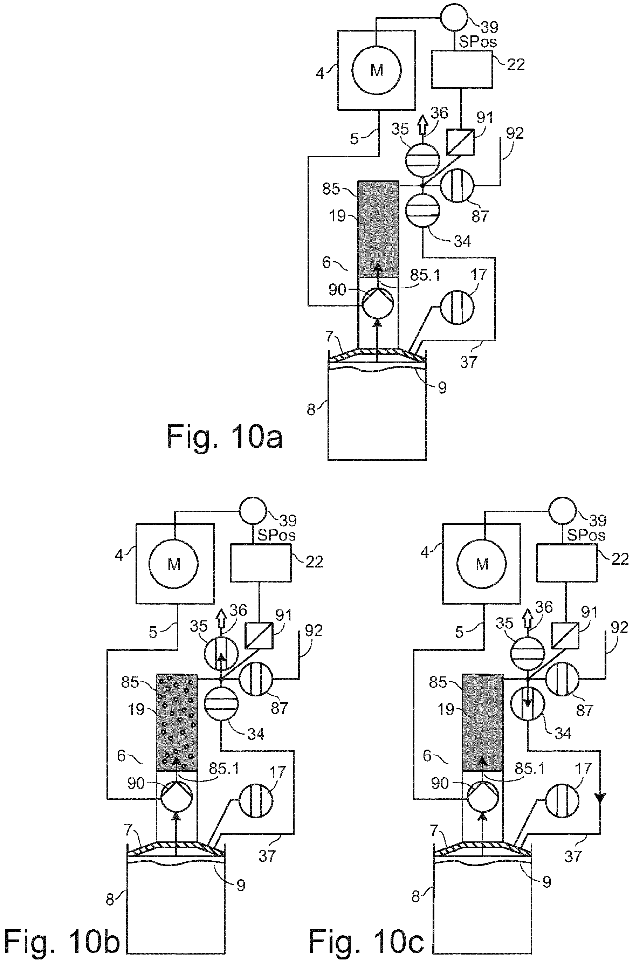

In den Figuren 10a bis 10c sind anhand von drei Blockschaltbildern drei verschiedene Betriebszustände einer zusätzlich möglichen Ausführungsform der Fördervorrichtung 2 dargestellt. Die weitere Ausführungsform der Fördervorrichtung 2 unterscheidet sich von der oben beschriebenen Ausführungsform gemäss Figur 9 dadurch, dass die Pumpe 6 nicht als Schöpfkolbenpumpe, sondern als Exzenter-Schneckenpumpe, Schraubenspindelpumpe, Spindelpumpe oder Zahnradpumpe ausgebildet ist. Die Pumpe 6 kann so betrieben werden, dass sie dauerhaft versucht Material 9 in die Kammer 85 zu fördern. Dazu kann die Pumpe 6 kontinuierlich mit einem Elektromotor, zum Beispiel mit einem Servomotor, angetrieben werden. Dadurch wird in der Kammer 85 ein definiertes Volumen eingestellt und ein Druck p aufgebaut, der über einen Drucksensor 91 erfasst wird. Die Pumpe 6 weist ein Pumpen-Stellglied 90 auf, das in der Messkammer 85 das Material-Volumen einstellt und damit in der Messkammer 85 den Druck p aufbaut. Das Pumpen-Stellglied 90 können bei einer Exzenter-Schneckenpumpe die Exzenter-Schnecke, bei einer Schraubenspindelpumpe die Schraubenspindel, bei einer Spindelpumpe die Spindel und bei einer Zahnradpumpe diejenigen Zähne sein, die in der Messkammer 85 den Druck p aufbauen beziehungsweise das Material-Volumen in der Messkammer 85 einstellen.

Vergleicht man eine blasenfreie Messprobe 19 mit einer Messprobe 19, die Luft enthält, stellt sich bei einer definierten Winkeländerung der Antriebswelle des Servomotors 4 in der Kammer 85 bei der luftbehafteten Messprobe 19 ein niedrigerer Druck P ein als bei einer blasenfreien Messprobe. Somit kann die Steuerung 22 aus dem Drehwinkel-Signal SPos, das den Drehwinkel der Antriebswelle des Servomotors 4 repräsentiert und dem Druck P in der Kammer 85 auf die Kompressibilität der Messprobe 19 schliessen. Der Drehwinkel der Antriebswelle des Servomotors 4 kann über den Weg-Sensor 39 erfasst werden. Wenn sich Luft in der Messprobe 19 befindet, wird ein vorgegebener Referenzdruck Pref, der einem Schwellwert gleicht, nicht erreicht. Befindet sich hingegen keine Luft in der Messprobe 19, wird der Referenzdruck Pref erreicht oder überschritten. Damit kann die Steuerung 22 die Entlüftung auf die oben beschriebenen Arten durchführen (siehe zum Beispiel den Abschnitt zur Ausführungsform gemäss Figur 8).

Der Entlüftungsmodus kann, wie bereits oben beschrieben, eine erste und eine zweite Entlüftungsphase umfassen. Um Wiederholungen zu vermeiden, wird bezüglich der Erläuterung der Entlüftungsphasen auf die obige Beschreibung verwiesen und insbesondere auf die Beschreibung betreffend die Ausführungsform gemäss Figur 7 und 8.

Wenn die Steuerung 22 durch Ermittlung der Kompressibilität der Messprobe 19 feststellt, dass sich blasenbehaftetes Material in der Messkammer 85 befindet (Figur 10b) wird, wie bei den anderen oben beschriebenen Ausführungsformen, das Ablassventil 35 geöffnet, um das Material zu entsorgen. Wenn die Steuerung 22 durch Ermittlung der Kompressibilität der Messprobe 19 hingegen feststellt, dass sich brauchbares Material in der Messkammer 85 befindet (Figur 10c) wird, wie bei den anderen oben beschriebenen Ausführungsformen auch, das Rückführventil 34 geöffnet, um das Material zurück in den Behälter 8 zu transportieren.

Mit der Fördervorrichtung 2 beziehungsweise 102 können verschiedene, viskose Materialien gefördert werden, zum Beispiel auch niedrigviskoses Silikon.

Wenn das Fördersystem 1 mit zwei Fördervorrichtungen 2 und 102 ausgestattet ist, können auch Zwei-Komponenten-Materialien, wie Zwei-Komponenten-Silikon, unter Druck einer nachfolgenden Mischeinheit 200 zugeführt werden. Die bis zum Mischer 200 getrennt geförderten Materialien können im Mischer 200 zum Beispiel im Verhältnis 1:1 gemischt werden. Bei Bedarf können den geförderten Materialien im Mischer 200 auch Additive zugemischt werden. Damit kann zum Beispiel Einfluss auf die Farbgestaltung oder die Materialeigenschaften genommen werden.

Vorzugsweise weist die Folgeplatte 7 der Fördervorrichtung 2 einen Durchlass 73 für die Rückführleitung 37 auf.

Es ist auch von Vorteil, wenn die Ventile 33, 34, 35 der Fördervorrichtung 2 als Nadelventile ausgebildet sind. Dies ist aber nicht zwingend notwendig. Eines oder mehrere der Ventile 33, 34, 35 kann auch als Kugelhahnventil ausgebildet sein.

Vorteilhafterweise beträgt das Volumen der Messkammer 31 weniger als 50 ccm. Die Messkammer 31 kann aber auch ein Volumen von 60 ccm aufweisen. Ein kleines Messkammervolumen ist von Vorteil, weil hiermit kleine Messproben gezogen werden können. Damit kann der Anteil des Ausschusses, also des als nicht blasenfrei klassifizierten Materials, noch weiter reduziert werden.

Die vorhergehende Beschreibung der Ausführungsbeispiele gemäss der vorliegenden Erfindung dient nur zu illustrativen Zwecken und nicht zum Zwecke der Beschränkung der Erfindung. Im Rahmen der Erfindung sind verschiedene Änderungen und Modifikationen möglich. So sind beispielsweise die verschiedenen in den Figuren 1 bis 8 gezeigten Komponenten des Fördersystems 1 beziehungsweise der Fördervorrichtungen 2 und 102 auch auf eine andere als in den Figuren gezeigte Weise miteinander kombinierbar.

Darüber hinaus kann bei der erfindungsgemässen Fördervorrichtung 2 eine Schnelllösevorrichtung vorgesehen sein, mit der die Messvorrichtung 30 an die Fördervorrichtung 2 beziehungsweise 102 anschliessbar und abnehmbar ist. Dies ist insbesondere von Vorteil, wenn mehrere Fördervorrichtungen vorhanden sind. Es genügt eine einzige Messvorrichtung 30, die schnell und einfach an die jeweilige Fördervorrichtung angeschlossen werden kann, um dort die Messung und gegebenenfalls die anschliessende Entlüftung durchzuführen. Die Schnelllösevorrichtung ist vorzugsweise so ausgebildet, dass die Messvorrichtung 30 werkzeuglos angeschlossen werden kann.

### Bezugszeichenliste

- 1: Fördersystem
- 2: Fördervorrichtung für Komponente A
- 4: Pumpenantrieb
- 5: Antriebsstange
- 6: Pumpe
- 7: Folgeplatte
- 8: Behälter
- 9: zu förderndes Material
- 10: Joch
- 11: Hubzylinder
- 13: Führung
- 14: Hubstange
- 15: Führung
- 16: Hubstange
- 17: Entlüftungsventil
- 18: Motorhalterung
- 19: Messprobe
- 20: Gehäuse
- 21: Transportrollen
- 22: Steuerung
- 23: Stange
- 30: Messvorrichtung
- 31: Messkammer
- 31.1: Messkammereinlass
- 32: Zylinder
- 33: Einlassventil
- 34: Auslass- oder auch Rückführventil
- 35: Ablassventil (zur Entsorgungsleitung)
- 36: Entsorgungsleitung
- 37: Material-Rückführleitung
- 38: Material-Zuführleitung
- 39: Weg-Sensor
- 40: Kolben
- 41: Messkammergehäuse
- 42: Kolbendichtung
- 43: T-Stück
- 45.1: Steueranschluss
- 45.2: Steueranschluss
- 46.1: Steueranschluss
- 46.2: Steueranschluss
- 47.1: Steueranschluss
- 47.2: Steueranschluss
- 48: Steueranschluss
- 50: Druckaufnehmer
- 51: Auslass Komponente A
- 52: Schleppkette
- 71: Dichtung
- 72: Öffnung
- 73: Durchlass
- 74: druckwirksame Fläche
- 81: Schöpfkolben
- 82: Schöpfkolbenteller
- 83: Pumpenkammer
- 84: Rückschlagventil
- 85: Pumpenkammer/Messkammer
- 85.1: Pumpenkammereinlass bzw. Messkammereinlass
- 86: Rückschlagventil
- 87: Förderventil
- 88: Kammer
- 90: Pumpen-Stellglied
- 91: Drucksensor
- 92: Material-Transportleitung
- 95: Kolben
- 96: Kolben
- 97: Splint
- 102: Fördervorrichtung für Komponente B
- 104: Pumpenantrieb
- 107: Folgeplatte
- 110: Joch
- 111: Hubzylinder
- 113: Führung
- 115: Führung
- 118: Motorhalterung
- 121: Transportrollen
- 150: Druckaufnehmer
- 151: Auslass Komponente B
- 152: Schleppkette
- 200: Mischeinheit
- DL: Druckluft
- SPos: Drehwinkel-Signal

## Patentansprüche

1. Fördervorrichtung zum Fördern eines viskosen Materials aus einem Behälter,
- bei der eine in den Behälter (8) einsetzbare Folgeplatte (7) und eine Pumpe (6) vorgesehen sind, mit der das viskose Material (9) durch die Folgeplatte (7) hindurch förderbar ist,
- bei der eine Messkammer (31; 85) zur Aufnahme einer Messprobe (19) des viskosen Materials (9) vorgesehen ist,
- bei der die Messkammer (31; 85) mit einer verschliessbaren Material-Einlassöffnung (31.1; 85.1) vorgesehen ist,
- bei der eine von der Messkammer (31; 85) wegführende verschliessbare Entsorgungsleitung (36) vorgesehen ist,
- bei der eine von der Messkammer (31; 85) über die Folgeplatte (7) in den Behälter (8) führende verschliessbare Material-Rückführleitung (37) vorgesehen ist,
- bei der eine Steuerung (22) vorgesehen ist, die derart ausgebildet und betreibbar ist, dass sie
-- bei mehreren Messproben (19) jeweils deren Kompressibilität ermittelt, und
-- abhängig von der ermittelten Kompressibilität, die Entsorgungsleitung (36) oder die Material-Rückführleitung (37) für die in der Messkammer (31; 85) befindliche Messprobe (19) freigibt.

2. Fördervorrichtung nach Patentanspruch 1,
- bei der ein Stellglied (40; 96) vorgesehen ist, mit dem ein bestimmter Druck in der Messkammer (31; 85) erzeugbar ist, und
- bei der ein Weg-Sensor (39) zur Ermittlung der Änderung des Volumens der Messkammer (31; 85) vorgesehen ist.

3. Fördervorrichtung nach Patentanspruch 1 oder 2,
bei der das Stellglied ein Kolben (40; 96) oder eine Membrane ist.

4. Fördervorrichtung nach Patentanspruch 1,
- bei der die Pumpe (6) ein Pumpen-Stellglied (90) aufweist, mit dem ein bestimmtes Volumen in der Messkammer (85) einstellbar ist, und
- bei der ein Druck-Sensor (91) zur Ermittlung des Drucks in der Messkammer (31; 85) vorgesehen ist.

5. Fördervorrichtung nach Patentanspruch 2, 3 oder 4, bei der der Weg-Sensor (39) vorgesehen ist, um die Position des Stellglieds (40; 96) zu erfassen.

6. Fördervorrichtung nach einem der Patentansprüche 2 bis 5,
bei der die Steuerung (22) das Signal des Weg-Sensors (39) nutzt, um die Kompressibilität der in der Messkammer (31) befindlichen Messprobe (19) zu ermitteln.

7. Fördervorrichtung nach einem der Patentansprüche 2 bis 6,
bei der der Weg-Sensor (39) als magnetostriktiver Sensor ausgebildet ist.

8. Fördervorrichtung nach einem der Patentansprüche 1 bis 7,
bei der die Messkammer (85) Teil der Pumpe (6) ist.

9. Fördervorrichtung nach einem der vorherigen Patentansprüche,
bei der die Pumpe (6) eine Kolbenpumpe, Zahnradpumpe, Spindelpumpe oder Exzenter-Schneckenpumpe ist.

10. Fördervorrichtung nach einem der vorherigen Patentansprüche,
bei der die Folgeplatte (7) ein Entlüftungsventil (17) aufweist.

11. Fördervorrichtung nach einem der vorherigen Patentansprüche,
bei der die Folgeplatte (7) eine druckwirksame Fläche (74) aufweist, die schräg verläuft.

12. Fördervorrichtung nach einem der vorherigen Patentansprüche,
bei der die Material-Rückführleitung (37) ein Rückführventil (34) aufweist.

13. Fördersystem mit einer ersten und einer zweiten Fördervorrichtung nach einem der vorherigen Patentansprüche 1 bis 12,
bei der die erste Fördervorrichtung (2) und die zweite Fördervorrichtung (102) ausgangsseitig mit einer Mischeinheit (200) verbunden sind.

14. Verfahren zum Betreiben einer Fördervorrichtung nach einem der vorherigen Patentansprüche 1 bis 12, das folgende Schritte umfasst:
- Material wird in die Messkammer (31; 85) gefördert,
- nachdem die Material-Einlassöffnung (31.1; 85.1), die Entsorgungsleitung (36) und die Material-Rückführleitung (37) geschlossen sind, wird die Kompressibilität des in der Messkammer (31; 85) befindlichen Materials (19) ermittelt,
- mittels einer Steuerung (22) wird abhängig von der ermittelten Kompressibilität, dafür gesorgt, dass das in der Messkammer (31) befindliche Material (19) über die Entsorgungsleitung (36) entsorgt oder über die Material-Rückführleitung (37) zurück in den Behälter (8) geführt wird.

15. Verwendung der Fördervorrichtung nach einem der vorherigen Patentansprüche 1 bis 12 zum Fördern eines viskosen Materials (9).

## Claims

1. A conveying device for conveying a viscous material from a container,
- in which a follower plate (7), which can be inserted into the container (8), and a pump (6) are provided, by means of which pump the viscous material (9) can be conveyed through the follower plate (7),
- in which a measuring chamber (31; 85) is provided for receiving a measuring sample (19) of the viscous material (9),
- in which the measuring chamber (31; 85) is provided with a closable material inlet opening (31.1; 85.1),
- in which a closable disposal line (36) is provided, which leads away from the measuring chamber (31; 85),
- in which a closable material return line (37) is provided, which leads from the measuring chamber (31; 85) into the container (8) via the follower plate (7),
- in which a controller (22) is provided, which is formed and can be operated in such a way that
-- in the case of several measuring samples (19), it determines the compressibility thereof in each case, and
-- it releases the disposal line (36) or the material return line (37) for the measuring sample (19) located in the measuring chamber (31; 85) as a function of the determined compressibility.

2. The conveying device according to claim 1,
- in which an actuator (40; 96) is provided, by means of which a certain pressure can be generated in the measuring chamber (31; 85), and
- in which a position sensor (39) for determining the change of the volume of the measuring chamber (31; 85) is provided.

3. The conveying device according to claim 1 or 2,
in which the actuator is a piston (40; 96) or a membrane.

4. The conveying device according to claim 1,
- in which the pump (6) has a pump actuator (90), by means of which a certain volume can be set in the measuring chamber (85), and
- in which a pressure sensor (91) is provided for determining the pressure in the measuring chamber (31; 85).

5. The conveying device according to claim 2, 3, or 4, in which the position sensor (39) is provided to detect the position of the actuator (40; 96).

6. The conveying device according to any one of claims 2 to 5,
in which the controller (22) uses the signal of the position sensor (39) to determine the compressibility of the measuring sample (19) located in the measuring chamber (31).

7. The conveying device according to any one of claims 2 to 6,
in which the position sensor (39) is formed as magneto-strictive sensor.

8. The conveying device according to any one of claims 1 to 7,
in which the measuring chamber (85) is part of the pump (6).

9. The conveying device according to any one of the preceding claims,
in which the pump (6) is a piston pump, gear pump, spindle pump, or eccentric screw pump.

10. The conveying device according to any one of the preceding claims,
in which the follower plate (7) has a venting valve (17) .

11. The conveying device according to any one of the preceding claims,
in which the follower plate (7) has a pressure-active area (74), which runs obliquely.

12. The conveying device according to any one of the preceding claims,
in which the material return line (37) has a return valve (34).

13. A conveying system comprising a first and a second conveying device according to any one of the preceding claims 1 to 12,
in which the first conveying device (2) and the second conveying device (102) are connected to a mixing unit (200) on the outlet side.

14. A method for operating a conveying device according to any one of the preceding claims 1 to 12, which comprises the following steps:
- material is conveyed into the measuring chamber (31; 85),
- after the material inlet opening (31.1; 85.1), the disposal line (36), and the material return line (37) are closed, the compressibility of the material (19) located in the measuring chamber (31; 85) is determined,
- as a function of the determined compressibility, it is ensured by means of a controller (22) that the material (19) located in the measuring chamber (31) is disposed of via the disposal line (36) or is guided back into the container (8) via the material return line (37).

15. Use of the conveying device according to any one of the preceding claims 1 to 12
for conveying a viscous material (9).

## Revendications

1. Dispositif de transport utilisé pour le transfert d'une matière visqueuse depuis un récipient,
- dans lequel une plaque suiveuse (7) insérable dans le récipient (8) et une pompe (6) permettant le transfert de la matière visqueuse (9) par la plaque suiveuse (7) sont prévues,
- dans lequel une chambre de mesure (31; 85) est prévue pour la collecte d'un échantillon de mesure (19) de la matière visqueuse (9),
- dans lequel la chambre de mesure (31; 85) est prévue avec un orifice d'admission de matière verrouillable (31.1; 85.1),
- dans lequel une conduite d'évacuation verrouillable (36), partant de la chambre de mesure (31; 85), est prévue,
- dans lequel une conduite de recyclage de matière verrouillable (37), allant de la chambre de mesure (31; 85) au récipient (8) en passant par la plaque suiveuse (7), est prévue,
- dans lequel est prévu un système de commande (22) qui est conçu et exploitable de manière
-- à déterminer, dans le cas de plusieurs échantillons de mesure (19), leur compressibilité respective et
-- à libérer, en fonction de la compressibilité déterminée, la conduite d'évacuation (36) ou la conduite de recyclage de matière (37) pour l'échantillon de mesure (19) qui se trouve dans la chambre de mesure (31; 85).

2. Dispositif de transport selon la revendication 1,
- dans lequel un actionneur (40; 96) permettant de générer une pression définie dans la chambre de mesure (31; 85) est prévu et
- dans lequel un capteur de course (39) est prévu pour déterminer la modification du volume de la chambre de mesure (31; 85).

3. Dispositif de transport selon la revendication 1 ou 2,
dans lequel l'actionneur est un piston (40; 96) ou une membrane.

4. Dispositif de transport selon la revendication 1,
- dans lequel la pompe (6) présente un actionneur de pompe (90) permettant de régler un volume défini dans la chambre de mesure (85) et
- dans lequel un capteur de pression (91) est prévu pour déterminer la pression dans la chambre de mesure (31; 85).

5. Dispositif de transport selon la revendication 2, 3 ou 4,
dans lequel le capteur de course (39) est prévu pour enregistrer la position de l'actionneur (40; 96).

6. Dispositif de transport selon l'une des revendications 2 à 5,
dans lequel le système de commande (22) utilise le signal du capteur de course (39) pour déterminer la compressibilité de l'échantillon de mesure (19) qui se trouve dans la chambre de mesure (31).

7. Dispositif de transport selon l'une des revendications 2 à 6,
dans lequel le capteur de course (39) est conçu comme capteur magnétostrictif.

8. Dispositif de transport selon l'une des revendications 1 à 7,
dans lequel la chambre de mesure (85) fait partie intégrante de la pompe (6).

9. Dispositif de transport selon l'une des revendications précédentes,
dans lequel la pompe (6) est une pompe à piston, une pompe à engrenages, une pompe à vis d'Archimède ou une pompe à vis excentrique.

10. Dispositif de transport selon l'une des revendications précédentes,
dans lequel la plaque suiveuse (7) présente une vanne de purge (17).

11. Dispositif de transport selon l'une des revendications précédentes,
dans lequel la plaque suiveuse (7) présente une surface soumise à une action de pression (74), qui est inclinée.

12. Dispositif de transport selon l'une des revendications précédentes,
dans lequel la conduite de recyclage de matière (37) présente une vanne de remise en circulation (34).

13. Système de transport comprenant un premier et un deuxième dispositif de transport selon l'une des revendications précédentes 1 à 12,
dans lequel le premier dispositif de transport (2) et le deuxième dispositif de transport (102) sont reliés côté sortie à une unité de mélange (200).

14. Procédé d'exploitation d'un dispositif de transport selon l'une des revendications précédentes 1 à 12, comprenant les étapes suivantes:
- la matière est transportée dans la chambre de mesure (31; 85),
- après la fermeture de l'orifice d'admission de matière (31.1, 85.1), de la conduite d'évacuation (36) et de la conduite de recyclage de matière (37), la compressibilité de la matière (19) qui se trouve dans la chambre de mesure (31; 85) est déterminée,
- un système de commande (22) permet de s'assurer, en fonction de la compressibilité déterminée, que la matière (19) qui se trouve dans la chambre de mesure (31) est évacuée par la conduite d'évacuation (36) ou est remise en circulation dans le récipient (8) par la conduite de recyclage de matière (37).

15. Utilisation du dispositif de transport selon l'une des revendications précédentes 1 à 12 pour le transfert d'une matière visqueuse (9).
